# EUROPÄISCHE PATENTSCHRIFT

(11) **EP 3 691 566 B1**
(45) Veröffentlichungstag und Bekanntmachung des Hinweises auf die Patenterteilung: **22.02.2023**
(21) Anmeldenummer: 18786242.0
(22) Anmeldetag: 02.10.2018
(51) Int. Cl.: A61C 7/36

(54) **VERFAHREN ZUR HERSTELLUNG EINER GEFÜHRTEN AUFBISSSCHIENE**
METHOD FOR PRODUCING A GUIDED BITE GUARD
PROCÉDÉ POUR FABRIQUER UNE GOUTTIÈRE DENTAIRE GUIDÉE

(30) Priorität: 02.10.2017 DE 102017217558
(43) Veröffentlichungstag der Anmeldung: 12.08.2020
(73) Patentinhaber: Sirona Dental Systems GmbH, 64625 Bensheim (DE)
(72) Erfinder: BÜHLER, Elias, 8049 Zürich (CH); OSKAM, Thomas, 8200 Schaffhausen (CH); MAKRIS, Evangelos, 8152 Glattbrugg (CH)
(74) Vertreter: Özer, Alpdeniz
(86) Internationale Anmeldenummer: PCT/EP2018/076780
(87) Internationale Veröffentlichungsnummer: WO 2019/068703

(56) Entgegenhaltungen:
- EP-A1- 1 516 604
- EP-B1- 1 516 604
- WO-A1-2011/131243
- WO-A1-2017/125799
- WO-A2-2012/140021
- DE-U1-202004 020 196
- US-A1- 2005 022 824
- US-A1- 2015 238 280

## Beschreibung

### Technisches Gebiet

Die Erfindung betrifft ein Verfahren zur Herstellung einer geführten Aufbissschiene für einen Auflagekiefer umfassend mindestens eine Führung für einen Gegenkiefer.

### Stand der Technik

Aus dem Stand der Technik sind Herstellungsverfahren einer geführten Aufbissschiene bekannt, bei denen eine geführte Aufbissschiene in einen Artikulator eingespannt wird und schrittweise angepasst wird. Dabei werden insbesondere die Führungsbahnen durch eine Artikulationsbewegung im Artikulator mechanisch schrittweise hergestellt, indem das Material manuell schrittweise abgetragen wird.

DE 20 2010 006 250 U1 offenbart eine Vorrichtung zur Herstellung einer Führungsschiene zur Korrektur der Kondylenposition eines Kiefergelenks, wobei die Vorrichtung eine lageveränderbare Plattform aufweist, auf der ein Unterkiefermodell angeordnet wird und relativ zum Oberkiefermodell in seiner Lage mittels einer Steuereinrichtung verändert wird.

DE 10 2013 112 032 A1 offenbart ein Verfahren zum Konstruieren einer Aufbissschiene, wobei 3D-Daten der beiden Kiefer erfasst werden und die Lage der Kiefer und der Kiefergelenke des Patienten relativ zueinander erfasst werden, wobei eine Okklusion simuliert wird, wobei dentale Störkontakte eines craniomandibulären Systems identifiziert werden und eine Aufbissschiene unter Verwendung der erfassten 3D-Daten und aus der Simulation der gewonnen Daten konstruiert wird, sodass die identifizierten Störkontakte eliminiert werden. Zur Vermeidung von Schmerzen wird eine Serie von mehreren Aufbissschienen zur interaktiven Annäherung an die physiologische Lage der Kiefergelenke konstruiert, wobei die Aufbissschienen der Serie in ihrer Form sich lediglich gering voneinander unterscheiden und somit die Kiefergelenke in kleinen diskreten Schritten über einen längeren Zeitraum hinweg in ihre physiologische Lage gebracht werden.

US 2015/0238280 A1 offenbart eine Vorrichtung und ein Verfahren zur Positionierung der Kiefer zur Kieferkorrektur, wobei ein Behandlungsplan bereitgestellt wird, wobei ein virtuelles Modell einer zahnärztlichen Vorrichtung eine erste Schale und eine zweite Schale aufweist, die so konfiguriert sind, dass sie die beiden Kiefer des Patienten neu positionieren. An der oberen Schale kann ein erstes Element und an der unteren Schale kann ein zweites Element angebracht sein, wobei die beiden Elemente in Kontakt zueinander kommen, um eine gewünschte Verschiebung der beiden Kiefer herbeizuführen.

EP 1516604 A1 offenbart ein intraorales Therapiegerät, vorzugsweise ein schneidig-Therapiegerät, mit einer Oberkiefers Schiene und einer Unterkiefers Schiene, wobei die beiden Schienen mit einem festen Stab gelenkig miteinander verbunden sind.

Ein Nachteil der bekannten Verfahren besteht darin, dass die Führungen einer geführten Aufbissschiene nachträglich durch ein manuelles Nachbearbeiten hinzugefügt werden.

Die Aufgabe der vorliegenden Erfindung besteht daher darin, ein Verfahren zur Herstellung einer geführten Aufbissschiene bereitzustellen, die eine präzise und zeitsparende Herstellung einer geführten Aufbissschiene erlaubt, wobei manuelle Herstellungsfehler, insbesondere der Führungen der Ausbissschiene, vermieden werden sollen.

### Darstellung der Erfindung

Die Erfindung betrifft ein Verfahren zur Herstellung einer geführten Aufbissschiene für einen Auflagekiefer umfassend mindestens eine Führung für einen Gegenkiefer. Ein 3D-Modell eines Oberkiefers und/oder ein 3D-Modell eines Unterkiefers sind bereits vorhanden, wobei die 3D-Modelle des Oberkiefers und des Unterkiefers in einer Okklusalstellung zueinander angeordnet werden. Unter Verwendung des 3D-Modells des Oberkiefers und/oder des 3D-Modells des Unterkiefers wird anschließend ein 3D-Modell der Aufbissschiene konstruiert, wobei am 3D-Modell der Aufbissschiene computergestützt automatisch die mindestens eine Führung für den Gegenkiefer konstruiert wird.

Die geführte Aufbissschiene wird auf dem Oberkiefer als einem Auflagekiefer und somit dem Unterkiefer als Gegenkiefer oder auf dem Unterkiefer als Auflagekiefer und somit dem Oberkiefer als Gegenkiefer konstruiert.

Eine geführte Aufbissschiene wird in der zahnärztlichen und kieferorthopädischen Behandlung von Zähneknirschen, Bruxismus und craniomandibulären Dysfunktionen aber auch als Mundschutz verwendet.

Eine geführte Aufbissschiene kann beispielsweise eine Michiganschiene sein, die eingesetzt wird, um Muskelschmerzen und Gelenkschmerzen sowie eine unsichere Schlussbisslage zu vermeiden. Die Michiganschiene wirkt vor allem als Relaxionsschiene. Die Führung des Unterkiefers auf der Aufbissschiene wird meistens im Eckzahnbereich durchgeführt.

Die geführte Aufbissschiene kann auch eine DROS-Aufbissschiene sein, die insbesondere zur mandibulären Stabilisierung dient.

Die geführte Aufbissschiene dient also als Relaxionsschiene, um okklusale Fehlfunktionen zu unterbinden und die Kaumuskulatur zu entspannen (Verminderung des Muskeltonus). Die geführte Ausbissschiene soll auch vor der Zerstörung der Zahnhartsubstanz schützen, die durch Zähneknirschen verursacht wird.

Eine geführte Aufbissschiene kann zur Behandlung unterschiedlicher Fehlbissstellungen, wie einem Fehlbiss mit einem zurückliegenden Unterkiefer oder einem Fehlbiss mit einem vorstehenden Unterkiefer, behandelt werden. Die Kieferanomalien können entweder angeboren oder erworben sein. Das Ausmaß des Fehlbisses wird erst beim Kieferschluss deutlich und zeigt sich beispielweise durch einen offenen Biss oder dadurch, dass die Oberkieferzähne hinter die Unterkieferzähne beißen statt umgekehrt. Die Position der Kiefer zueinander und zum Gesichtsschädel hat einen entscheidenden Einfluss auf das Gesichtsprofil. In Abhängigkeit davon, ob die Überentwicklung oder Unterentwicklung im Oberkiefer und/oder im Unterkiefer lokalisiert ist resultieren parallel zum Fehlbiss auch unterschiedliche Gesichtsprofile.

Die Führung der Aufbissschiene wird meistens im Frontzahnbereich und/oder im Eckzahnbereich angeordnet und kann einen bestimmten Neigungswinkel relativ zu einer Einschubachse der Aufbissschiene zwischen 20 und 40 Grad üblicherweise in lateraler Richtung und/oder protrusaler Richtung einer Kieferbewegung aufweisen.

Das 3D-Modell des Oberkiefers und/oder des Unterkiefers sind zur Durchführung des Verfahrens bereits vorhanden, und wurden beispielweise mittels einer intraoralen 3D-Kamera vermessen.

Die 3D-Modelle der beiden Kiefer werden in der Okklusalstellung zueinander angeordnet und in einen virtuellen Artikluatormodell integriert. Zur Anordnung der beiden 3D-Modelle relativ zueinander kann beispielsweise eine seitliche 3D-Aufnahme der beiden Kiefer des Patienten in der Schlussbissstellung verwendet werden.

Die mindestens eine Führung für den Gegenkiefer wird computergestützt automatisch konstruiert, wobei in der Schlussbissstellung die Führung der Aufbissschiene eine Kraft in eine gewünschte Richtung auf den Gegenkiefer zur Korrektur der Fehlbissstellung ausübt.

Bei einem vorstehenden Unterkiefer soll der Unterkiefer zur Korrektur des Fehlbisses in eine distale Richtung zum Ende des Zahnbogens verschoben werden. Bei einem rückliegenden Unterkiefer soll der Unterkiefer in eine mesiale Richtung zur Mitte des Zahnbogens hin verschoben werden.

Ein Vorteil des vorliegenden Verfahrens besteht darin, dass die geführte Aufbissschiene virtuell computergestützt konstruiert wird und nach dem konstruierten 3D-Modell der geführten Aufbissschiene mittels eines CAD/CAM-Verfahrens oder mittels eines 3D-Druckers die geführte Aufbissschiene vollautomatisch hergestellt werden kann.

Ein weiterer Vorteil des vorliegenden Verfahrens besteht darin, dass die mindestens eine Führung der geführten Aufbissschiene exakt positioniert werden kann und präzise an den Gegenkiefer angepasst werden kann. Das aufwendige schrittweise Abtragen des Materials zur Herstellung der Führungen wird dabei im Vergleich zu den bekannten Herstellungsverfahren eingespart.

Vorteilhafterweise können die 3D-Modelle des Oberkiefers und des Unterkiefers in einem virtuellen Artikulatormodell integriert werden, das eine Artikulationsbewegung des Unterkiefers relativ zum Oberkiefer simuliert.

Im virtuellen Artikulatormodell wird ein Artikulator simuliert, der die Kiefergelenksbewegung nachahmt. Die Bewegung der Kiefer zueinander wird dabei simuliert, wobei das Kiefergelenk sowohl Drehbewegungen (Öffnungs-und Schließbewegungen um die Gelenkachse), als auch Gleitbewegungen (Vorschubsbewegungen) ausführen kann. Die Kiefergelenke bestehen aus einem knorpelüberzogenen Gelenkköpfchen, einer ebenfalls knorpelüberzogenen Gelenkgrube und der faserknorpeligen Zwischengelenkscheibe. Ferner besteht das Kiefergelenk aus weichgewebigen Strukturen, wie dem Bandapparat, Gefäßen und Nerven. Bei der Mundöffnung gleitet das Gelencköpfchen zusammen mit dem Diskus, der im Normalfall fest mit dem Gelenkköpfchen verbunden ist nach vorne und unten. Bei allen Bewegungen, die der Unterkiefer ausführt, sind die Kiefergelenke und die Kaumuskulatur aktiv. Diese Bewegungen sollen mittels des virtuellen Artikulatormodells imitiert werden. Zur Erzeugung des virtuellen Aritkulatormodells können beispielsweise die relativen Positionen der beiden Kiefer zueinander für unterschiedliche Öffnungswinkel gemessen werden und entsprechend auf das virtuelle Artikulatormodell übertragen werden.

Die geführte Aufbissschiene wird dann unter Verwendung der beiden 3D-Modelle der Kiefer und unter Berücksichtigung der simulierten Bewegungen der Kiefergelenke im virtuellen Artikulatormodell computergestützt konstruiert.

Alternativ zum virtuellen Artikulatormodell können die Bewegungen der beiden Kiefer zueinander auch durch sogenannte Heuristiken berechnet bzw. simuliert werden, beispielsweise eine translatorische Bewegung der beiden 3D-Modelle der Kiefer zueinander.

Vorteilhafterweise kann die Ausdehnung der geführten Aufbissschiene auf dem Oberkiefer bzw. auf dem Unterkiefer als Auflagekiefer computergestützt automatisch oder durch einen Benutzer festgelegt werden.

Dadurch wird die Ausdehnung der geführten Aufbissschiene relativ zum Auflagekiefer festgelegt. Die Ausdehnung kann dabei unter Verwendung eines virtuellen Werkzeugs durch den Benutzer festgelegt werden, indem die Grenze der geführten Aufbissschiene auf dem 3D-Modell des Auflagekiefers eingezeichnet wird. Alternativ kann die Ausdehnung der Aufbissschiene automatisch computergestützt festgelegt werden, indem auf eine Datenbank unterschiedlicher 3D-Modelle von Auflagekiefern zugegriffen wird und ein passendes 3D-Modell eines Auflagekiefers ausgewählt wird für das bereits die Ausdehnung der Aufbissschiene definiert ist.

Vorteilhafterweise kann eine minimale Dicke der geführten Aufbissschiene computergestützt automatisch oder durch einen Benutzer festgelegt werden.

Dadurch kann die minimale Dicke der Aufbissschiene festgelegt werden. Der Benutzer kann beispielweise den Wert für die minimale Dicke manuell eingeben. Alternativ kann die minimale Dicke computergestützt automatisch festgelegt werden, indem das vermessene 3D-Modell des Auflagekiefers mit der Datenbank unterschiedlicher Auflagekiefer verglichen wird, für die minimalen Dicken bereits festgelegt wurden. Die geführte Aufbissschiene muss dabei überall die vorgegebene minimale Dicke entlang einer Einschubachse der Aufbissschiene aufweisen. Die Einschubachse wird durch eine Einschubrichtung beim Aufsetzen der Aufbissschiene auf den Auflagekiefer definiert. Die vorgegebene minimale Dicke kann jedoch zum Rand der Aufbissschiene unterschritten werden.

Vorteilhafterweise kann ein definierter Abstand einer Okklusionsöffnung in einer gewünschten Okklusalstellung der geführten Aufbissschiene computergestützt automatisch oder durch einen Benutzer festgelegt werden.

Dadurch wird der definierte Abstand der Okklusionsöffnung definiert. Der Abstand kann beispielsweise durch einen Benutzer eingegeben werden oder computergestützt automatisch durch den Vergleich mit einer Datenbank unterschiedlicher Auflagekiefer und Gegenkiefer mit bekannten Okklusionsöffnungen verglichen werden. Der Abstand der Okklusionsöffnung kann beispielsweise durch den Abstand zwischen den entsprechenden Okklusionskontakten des Auflagekiefers und des Gegenkiefer definiert werden.

Das 3D-Modell der geführten Aufbissschiene wird computergestützt automatisch berechnet, wobei die Bedingungen zu erfüllen sind, dass die geführte Aufbissschiene einen definierten Auflagebereich auf dem Auflagekiefer abdeckt, dass für mindestens einen Zahn des Gegenkiefers mindestens eine lokale Höckerspitze als Auflagepunkt definiert wird.

Dadurch wird das 3D-Modell der geführten Aufbissschiene computergestützt automatisch berechnet, wobei die Bedingungen erfüllt werden. Der definierte Auflagebereich kann dabei vorher manuell durch einen Benutzer oder computergestützt automatisch durch den Vergleich mit einer Datenbank unterschiedlicher Auflagekiefer ermittelt werden. Durch den definierten Auflagepunkt für die einzelnen Zähne wird eine stabile mechanische Auflage der Aufbissschiene auf dem Auflagekiefer gewährleistet.

Vorteilhafterweise kann mindestens ein Führungspunkt der zu konstituierenden Führung auf dem Gegenkiefer computergestützt automatisch oder manuell durch einen Benutzer definiert werden.

Dadurch wird der Führungspunkt manuell mittels eines virtuellen Werkzeugs durch den Benutzer oder computergestützt automatisch durch den Vergleich mit einer Datenbank unterschiedlicher Auflagekiefer und Gegenkiefer mit festgelegten Führungspunkten definiert.

Vorteilhafterweise kann für jeden Führungspunkt mindestens ein Bewegungsprofil definiert werden.

Für jeden Führungspunkt wird also ein Bewegungsprofil beispielsweise mittels eines virtuellen Werkzeugs durch den Benutzer festgelegt. Das Bewegungsprofil ist dabei als eine Funktion der Kieferöffnung in Abhängigkeit von einer Kiefer-Bewegungsdistanz definiert. Das Bewegungsprofil legt also einen gewünschten Bewegungsverlauf des Gegenkiefers bei Verringerung der Kieferöffnung bis zu einer geschlossenen Okklusalstellung, bei der der Gegenkiefer auf die Aufbissschiene beißt. Für den gewünschten Bewegungsverlauf wird also beispielsweise bei einem vorstehenden Unterkiefer eine Kraft auf den Unterkiefer in eine distale Richtung und bei einem zurückliegenden Unterkiefer eine Kraft in eine mesiale Richtung ausgeübt. Das Bewegungsprofil geht dabei durch eine festgelegte Ebene, die durch den Führungspunkt geht und beispielweise entlang einer Richtung der Einschubachse verläuft. Für einen Führungspunkt können auch mehrere Bewegungsprofile in unterschiedlichen Ebenen definiert werden.

Vorteilhafterweise kann in Abhängigkeit vom mindestens einem Bewegungsprofil die Oberflächenform der Führung computergestützt automatisch berechnet werden.

Dadurch wird die Oberflächenform der Führung in Abhängigkeit von mindestens einem festgelegten Bewegungsprofil computergestützt automatisch berechnet. Dabei kann die Oberfläche des 3D-Modells der geführten Aufbissschiene in den Bereichen der Führungen entsprechend angepasst beziehungsweise geführt werden, wobei ein Zwischenraum zwischen den festgelegten Bewegungsprofilen so interpoliert werden kann, dass ein glatter Übergang der Oberflächenform der Führung entsteht. Die Oberflächenform der Führung wird also berechnet, dass die konstruierte Führung zu dem gewünschten Bewegungsverlauf des Gegenkiefers relativ zur Aufbissschiene und relativ zum Auflagekiefer entsprechend den definierten Bewegungsprofilen führt.

Vorteilhafterweise können für jeden Führungspunkt mindestens zwei Bewegungsprofile in zwei unterschiedlichen Ebenen definiert werden, wobei die Oberflächenform der Führung zwischen den beiden Ebenen der beiden Bewegungsprofile so interpoliert wird, dass ein glatter Übergang entsteht.

Durch die Interpolation zwischen den Bewegungsprofilen entsteht also ein glatter Übergang der Oberflächenform der Führung, wobei durch die konstruierte Führung der gewünschte Bewegungsverlauf des Gegenkiefers relativ zum Auflagekiefer erreicht wird.

Eine erste Ebene eines ersten Bewegungsprofils kann beispielsweise in einer lateralen (seitlichen) Bewegungsrichtung und eine zweite Ebene eines zweiten Bewegungsprofils kann beispielsweise in einer protrusionalen (vor- und zurück) Bewegungsrichtung angeordnet sein.

Vorteilhafterweise kann bei der Konstruktion der Aufbissschiene die Oberfläche des virtuellen 3D-Modells der geführten Aufbissschiene soweit reduziert werden, dass kein Oberflächenpunkt des 3D-Modells des Gegenkiefers virtuell durchdrungen wird, solange der Gegenkiefer entlang der mindestens eine Führung innerhalb des virtuellen Artikulatormodells innerhalb eines definierten Bewegungsbereichs bewegt wird.

Dadurch wird das 3D-Modell der geführten Aufbissschiene soweit reduziert, dass die geführte Aufbissschiene lediglich an den definierten Führungen und in der definierten Okklusalstellung an den definierten Kontaktbereichen mit dem Gegenkiefer in Kontakt kommt, sodass störende Bereiche der Aufbissschiene reduziert beziehungsweise entfernt werden. Dadurch ist also eine nachträgliche manuelle Bearbeitung der Aufbissschiene zur Reduktion der Störbereiche nicht notwendig.

Vorteilhafterweise kann die geführte Aufbissschiene nach dem konstruierten 3D-Modell der Aufbissschiene vollautomatisch mittels eines subtraktiven Herstellungsverfahrens, wie einer CAM-Herstellungsmaschine, oder mittels eines additiven Herstellungsverfahrens, wie eines 3D-Druckers, hergestellt werden.

Dadurch wird die geführte Aufbissschiene nach dem konstruierten 3D-Modell vollautomatisch hergestellt.

Bei der Herstellung mittels einer CAM-Herstellungsmaschine wird ein Rohling in die CAM-Herstellungsmaschine eingespannt und mittels Fräswerkzeugen und/oder Schleifwerkzeugen so lange bearbeitet, bis die geführte Aufbissschiene nach dem konstruierten 3D-Modell hergestellt ist. Bei der Verwendung eines 3D-Druckers wird die konstruierte Aufbissschiene ausgedruckt. Der 3D-Drucker kann beispielsweise auf einem SLS-Verfahren (Selektives Lasersintern) beruhen, wobei das Drucken von dreidimensionalen Objekten ohne Bindemittel oder zusätzliche Montageschritte ermöglicht wird. Dabei wird das vorliegende 3D-Modell der Aufbissschiene mittels einer speziellen Slicing-Software in viele horizontale Ebenen zerlegt und als Steuerbefehle an den 3D-Drucker weitergeleitet. Der 3D-Drucker druckt das Objekt dann Schicht für Schicht aus, wobei einzelne Puderpartikel in einem Puderbett durch eine hohe Temperatur eines Lasers miteinander verschmolzen werden. Anschließend wird das Objekt abgesenkt und eine neue Pulverschicht aufgetragen. Der Vorgang wird so lange wiederholt, bis die gesamte geführte Aufbissschiene vollständig ausgedruckt ist. Der 3D-Drucker kann auch auf einem Stereo-Lithographie-Verfahren beruhen, wobei ein Laser eingesetzt wird, der eine aus lichtempfindlichem Harz und Materialpartikeln zusammengesetzte Masse polymerisiert. Das Material der geführten Aufbissschiene kann beispielsweise ein Kunststoff mit einer passenden Härte sein. Die Härte und die Elastizität des Kunststoffs werden dabei so gewählt, dass eine präzise Führung des Gegenkiefers entlang der konstruierten Führungen ermöglicht wird. Die Aufbissschiene kann auch aus unterschiedlichen Kunststoffen hergestellt werden.

Ein weiterer Gegenstand der Erfindung ist die geführte Aufbissschiene hergestellt unter Verwendung des oben genannten Verfahrens, wobei die mindestens eine Führung für den Gegenkiefer computergestützt automatisch konstruiert ist.

Der Vorteil einer solchen geführten Aufbissschiene besteht darin, dass die Führungen exakt positioniert werden, um einen definierten Bewegungsverlaufs relativ zum Auflagekiefer beziehungsweise zur Aufbissschiene zu gewährleisten.

Vorteilhafterweise kann die Führung für den Gegenkiefer unter Verwendung des mindestens einen Bewegungsprofils an mindestens einem Führungspunkt konstruiert sein.

Dadurch wird die Führung unter Verwendung des definierten Bewegungsprofils konstruiert, sodass die Führung zu dem gewünschten Bewegungsverlauf des Gegenkiefers führt.

Vorteilhafterweise kann die geführte Aufbissschiene nach dem konstruierten 3D-Modell der Aufbissschiene vollautomatisch mittels eines subtraktiven Herstellungsverfahrens, wie einer CAM-Herstellungsmaschine, oder mittels eines additiven Herstellungsverfahrens, wie eines 3D-Druckers, hergestellt sein.

Dadurch wird die geführte Aufbissschiene vollautomatisch und damit zeitsparend hergestellt, wobei mögliche Herstellungsfehler einer manuellen Herstellung der Führungen vermieden werden.

### Kurzbeschreibung der Zeichnungen

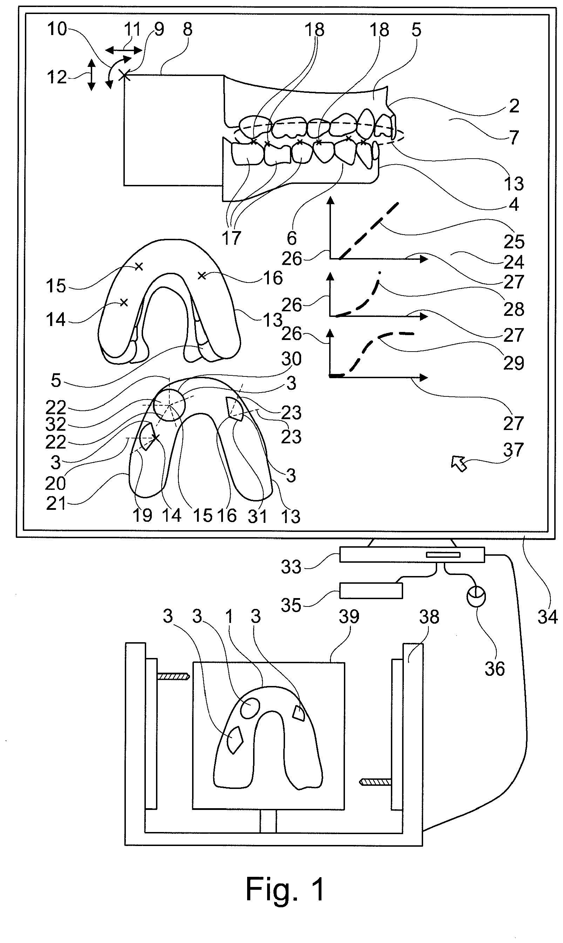

Die Erfindung wird anhand der Zeichnungen erläutert. Es zeigt, die
- Fig. 1: eine Skizze zur Verdeutlichung des Verfahrens zur Herstellung einer geführten Aufbissschiene.

### Ausführungsbeispiele

Die Fig. 1 zeigt eine Skizze zur Verdeutlichung des Verfahrens zur Herstellung einer geführten Aufbissschiene 1 für einen Auflagekiefer 2, im vorliegenden Fall für den Oberkiefer, umfassend mindestens eine Führung 3. Im vorliegenden Fall weist die Aufbissschiene 1 mehrere Führungen 3 mit dem Gegenkiefer 4, im vorliegenden Fall für den Unterkiefer, auf. Ein 3D-Modell 5 des Oberkiefers und ein 3D-Modell 6 des Unterkiefers sind bereits vorhanden und wurden mittels einer intraoralen 3D-Kamera am Patienten vermessen. Das 3D-Modell 5 des Oberkiefers und das 3D-Modell 6 des Unterkiefers sind in einer Okklusalstellung 7 zueinander angeordnet und in einem virtuellen Artikulatormodell 8 integriert. Das Artikulatormodell 8 simuliert dabei eine Artikulationsbewegung des Kiefergelenks 9, das zur Verdeutlichung als ein Kreuz dargestellt ist, und damit des Unterkiefers 6 relativ zum Oberkiefer 5. Die Artikulationsbewegung des Kiefergelenks 9 kann dabei aus einer Rotationsbewegung 10, einer Vorschubbewegung 11 in horizontaler Richtung, einer Bewegung 12 in vertikaler Richtung relativ zum 3D-Modell 5 des Oberkiefers und einer nicht dargestellten Seitwärtsbewegung in einer Drehachsenrichtung des Kiefergelenks 9 zusammengesetzt sein. Anschließend wird unter Verwendung des 3D-Modells 5 des Oberkiefers 2, des 3D-Modells 6 des Unterkiefers 4 ein 3D-Modell 13 der geführten Aufbissschiene 13 konstruiert, wobei der simulierte Bewegungsverlauf des Unterkiefers 4 relativ zum Oberkiefer 2 aus dem Artikulatormodell 8 verwendet wird. Anschließend werden am 3D-Modell 13 der Aufbissschiene 1 ein erster Führungspunkt 14, ein zweiter Führungspunkt 15 und ein dritter Führungspunkt 16 manuell durch einen Benutzer oder computergestützt automatisch festgelegt. Die Führungspunkte 14, 15 und 16 am 3D-Modell 13 der Aufbissschiene werden dabei in Relation zu ihren Positionen auf dem Gegenkiefer 4 festgelegt. Die Ausdehnung des 3D-Modells 13 der Aufbissschiene 1 relativ zum 3D-Modell 5 des Auflagekiefers 2 wird dabei manuell durch den Benutzer oder computergestützt automatisch so festgelegt, dass zumindest ein definierter Auflagebereich des Auflagekiefers 2 abgedeckt ist und eine Öffnung des Kiefers ein definierten Wert aufweist. Das 3D-Modell 13 der Aufbissschiene 1 wird so konstruiert, dass für jeden Zahn 17 des Gegenkiefers 4 eine lokale Höckerspitze als ein Auflagepunkt 18 definiert wird. Die Auflagepunkte 18 sind als Kreuze auf den Gegenzähnen 17 dargestellt. Für den ersten Führungspunkt 14 wird ein erstes Bewegungsprofil 19 in einer ersten Ebene ein zweites Bewegungsprofil 20 in einer zweiten Ebene und ein drittes Profil 21 in einer dritten Ebene festgelegt. Für den zweiten Führungspunkt 15 werden ebenfalls mehrere Bewegungsprofile 22 in unterschiedlichen Ebenen festgelegt. Für den dritten Führungspunkt 16 werden ebenfalls zwei Bewegungsprofile 23 in unterschiedlichen Ebenen festgelegt. In einem ersten Diagramm 24 ist ein Beispiel für eine erste Funktion 25 des erstes Bewegungsprofils 19 als Funktion einer Öffnung 26 des Gegenkiefers 4, nämlich ein Abstand der Kiefer zueinander, in Abhängigkeit von einem Abstand 27 des jeweiligen gleitenden Kontaktpunktes des Gegenkiefers in der Führung relativ zum jeweiligen Führungspunkt in der Ebene des jeweiligen Bewegungsprofils aufgetragen. Die erste Funktion 25 für das erste Bewegungsprofil 19 weist dabei einen linearen Verlauf auf. Eine zweite Funktion 28 des zweiten Bewegungsprofils 20 weist einen exponentialförmigen Verlauf auf. Eine dritte Funktion 29 des dritten Bewegungsprofils 21 weist einen S-förmigen Verlauf auf. In Abhängigkeit von den Bewegungsprofilen 19, 20 und 21 des ersten Führungspunktes 14 wird computergestützt automatisch die Oberflächenform der ersten Führung 3 berechnet. In Abhängigkeit von den fünf Bewegungsprofilen 22 des zweiten Führungspunktes 15 wird die zweite Führung 30 berechnet. In Abhängigkeit von den Bewegungsprofilen 23 des dritten Führungspunktes 16 wird die dritte Führung 31 berechnet. Dabei wird die Oberflächenform der Führung 3, 30, 31 in einem Zwischenbereich 32 zwischen benachbarten Bewegungsprofilen interpoliert, sodass ein glatter Übergang der Oberflächenform der Führung entsteht. Die Konstruktion der geführten Aufbissschiene erfolgt virtuell mittels eines Computers 33, wobei an den Computer 33 eine Anzeigevorrichtung 34, wie ein Monitor, sowie Bedienungselemente, wie eine Tastatur 35 und eine Maus 36, angeschlossen sind. Die Konstruktion des 3D-Modells 13 der Aufbissschiene 1 kann computergestützt automatisch und/oder manuell durch einen Benutzer unter Verwendung eines virtuellen Werkzeugs 37, wie beispielsweise eines Cursors, erfolgen. Nach der vollständigen Konstruktion des 3D-Modells 13 der Aufbissschiene 1 werden die Konstruktionsdaten an eine CAM-Herstellungsmaschine 38 weitergeleitet. In der CAM-Herstellungsmaschine 38 wird dann die herzustellende geführte Aufbissschiene 1 mit den Führungen 3 aus einem Rohling 39, der aus einem besonderen Kunststoff hergestellt sein kann, herausgeschliffen. Alternativ kann die geführte Aufbissschiene 1 auch mittels eines 3D-Druckers hergestellt werden.

### Bezugszeichen

- 1: Aufbissschiene
- 2: Auflagekiefer
- 3: Führungen
- 4: Gegenkiefer
- 5: 3D-Modell Oberkiefers
- 6: 3D-Modell des Unterkiefers
- 7: Okklusalstellung
- 8: Artikulatormodell
- 9: Kiefergelenk
- 10: Rotationsbewegung
- 11: Vorschubbewegung
- 12: Bewegung in vertikaler Richtung
- 13: 3D-Modell der Aufbissschiene
- 14: erster Führungspunkt
- 15: zweiter Führungspunkt
- 16: dritter Führungspunkt
- 17: Zähne
- 18: Auflagepunkte
- 19: erstes Bewegungsprofil
- 20: zweites Bewegungsprofil
- 21: drittes Bewegungsprofil
- 22: Bewegungsprofile
- 23: Bewegungsprofile
- 24: Diagramm
- 25: Funktion des ersten Bewegungsprofils
- 26: Öffnung des Gegenkiefers
- 27: Abstand des Kontaktpunktes
- 28: Funktion des zweiten Bewegungsprofils
- 29: Funktion des dritten Bewegungsprofils
- 30: zweite Führung
- 31: dritte Führung
- 32: Zwischenbereich
- 33: Computer
- 34: Anzeigevorrichtung
- 35: Tastatur
- 36: Maus
- 37: Cursor
- 38: CAM-Herstellungsmaschine
- 39: Rohling

## Patentansprüche

1. Verfahren zur Herstellung einer geführten Aufbissschiene (1) für einen Auflagekiefer (2) umfassend mindestens eine Führung (3, 30, 31) für einen Gegenkiefer (4), wobei ein 3D-Modell (5) eines Oberkiefers (2) und ein 3D-Modell (6) eines Unterkiefers (4) vorhanden sind, wobei die 3D-Modelle (5, 6) des Oberkiefers und des Unterkiefers in einer Okklusalstellung (7) zueinander angeordnet werden, wobei unter Verwendung des 3D-Modells (5) des Oberkiefers (2) und des 3D-Modells (6) des Unterkiefers (4) ein virtuelles 3D-Modell (13) der Aufbissschiene (1) konstruiert wird, wobei am 3D-Modell (13) der Aufbiss- schiene (1) computergestutzt automatisch die mindestens eine Führung (3, 30, 31) für den Gegenkiefer (4) konstruiert wird, **dadurch gekennzeichnet, dass** das 3D-Modell (13) der geführten Aufbissschiene (1) computergestützt automatisch berechnet wird, wobei die Bedingungen zu erfüllen sind, dass die geführte Aufbissschiene (1) einen definierten Auflagebereich auf dem Auflagekiefer (2) abdeckt, dass für mindestens einen Zahn (17) des Gegenkiefers (4) mindestens eine lokale Höckerspitze als Auflagepunkt (18) definiert wird.

2. Verfahren nach Anspruch 1, **dadurch gekennzeichnet, dass** die 3D-Modelle (5, 6) des Oberkiefers und des Unterkiefers in einem virtuellen Artikulatormodell (8) integriert werden, das eine Artikulationsbewegung (10, 11, 12) des Unterkiefers (4) relativ zum Oberkiefer (2) simuliert.

3. Verfahren nach Anspruch 1, **dadurch gekennzeichnet, dass** die Ausdehnung der geführten Aufbissschiene (1) auf dem Oberkiefer (2) bzw. auf dem Unterkiefer als Auflagekiefer (2) computergestützt automatisch oder durch einen Benutzer festgelegt wird.

4. Verfahren nach einem der Ansprüche 1-3, **dadurch gekennzeichnet, dass** eine minimale Dicke der geführten Aufbissschiene (1) computergestützt automatisch oder durch einen Benutzer festgelegt wird.

5. Verfahren nach einem der Ansprüche 1-4, **dadurch gekennzeichnet, dass** ein definierter Abstand einer Okklusionsöffnung in einer gewünschten Okklusalstellung (7) der geführten Aufbissschiene (1) computergestützt automatisch oder durch einen Benutzer festgelegt wird.

6. Verfahren nach einem der Ansprüche 1-5, **dadurch gekennzeichnet, dass** mindestens ein Führungspunkt (14, 15, 16) der zu konstituierenden Führung (3, 30, 31) auf dem Gegenkiefer (4) computergestützt automatisch oder manuell durch einen Benutzer definiert wird.

7. Verfahren nach Anspruch 6, **dadurch gekennzeichnet, dass** für jeden Führungspunkt (14, 15, 16) mindestens ein Bewegungsprofil (19, 20, 21, 22, 23) definiert wird.

8. Verfahren nach Anspruch 7, **dadurch gekennzeichnet, dass** in Abhängigkeit vom mindestens einen Bewegungsprofil (19, 20, 21, 22, 23) die Oberflächenform der Führung (3, 30, 31) computergestützt automatisch berechnet wird.

9. Verfahren nach Anspruch 8, **dadurch gekennzeichnet, dass** für jeden Führungspunkt (14, 15, 16) mindestens zwei Bewegungsprofile (19, 20, 21, 22, 23) in zwei unterschiedlichen Ebenen definiert werden, wobei die Oberflächenform der Führung (3, 30, 31) zwischen den beiden Ebenen der beiden Bewegungsprofile (19, 20, 21, 22, 23) so interpoliert wird, dass ein glatter Übergang entsteht.

10. Verfahren nach einem der Ansprüche 1-9, **dadurch gekennzeichnet, dass** bei der Konstruktion der Aufbissschiene (1) die Oberfläche des virtuellen 3D-Modells (13) der geführten Aufbissschiene (1) soweit reduziert wird, dass kein Oberflächenpunkt des 3D-Modells (6) des Gegenkiefers (4) virtuell durchdrungen wird, solange der Gegenkiefer (4) entlang der mindestens einen Führung (3) innerhalb des virtuellen Artikulatormodells (8) innerhalb eines definierten Bewegungsbereichs bewegt wird.

11. Verfahren nach einem der Ansprüche 1-10, **dadurch gekennzeichnet, dass** die geführte Aufbissschiene (1) nach dem konstruierten 3D-Modell (13) der Aufbissschiene (1) vollautomatisch mittels eines subtraktiven Herstellungsverfahrens, wie einer CAM-Herstellungsmaschine (38), oder mittels eines additiven Herstellungsverfahrens, wie eines 3D-Druckers, hergestellt wird.

## Claims

1. A method for producing a guided bite guard (1) for a supporting jaw (2) comprising at least one guide (3, 30, 31) for an opposing jaw (4), wherein a 3D model (5) of an upper jaw (2) and a 3D model (6) of a lower jaw (4) are present, wherein the 3D models (5, 6) of the upper jaw and of the lower jaw are arranged in an occlusal position (7) relative to one another, wherein the 3D model (5) of the upper jaw (2) and the 3D model (6) of the lower jaw (4) are used to construct a virtual 3D model (13) of the bite guard (1), wherein the at least one guide (3, 30, 31) for the opposing jaw (4) is constructed automatically in a computer-aided manner on the 3D model (13) of the bite guard (1), **characterised in that** the 3D model (13) of the guided bite guard (1) is automatically calculated in a computer-aided manner, wherein the conditions to be met are that the guided bite guard (1) covers a defined support region on the supporting jaw (2), that for at least one tooth (17) of the opposing jaw (4) at least one local cusp tip is defined as a support point (18).

2. The method according to claim 1, **characterised in that** the 3D models (5, 6) of the upper jaw and of the lower jaw are integrated in a virtual articulator model (8), which simulates an articulation movement (10, 11, 12) of the lower jaw (4) relative to the upper jaw (2).

3. The method according to claim 1, **characterised in that** the expansion of the guided bite guard (1) on the upper jaw (2) or on the lower jaw as the supporting jaw (2) is determined automatically in a computer-aided manner or by a user.

4. The method according to any of claims 1-3, **characterised in that** a minimum thickness of the guided bite guard (1) is determined automatically in a computer-aided manner or by a user.

5. The method according to any of claims 1-4, **characterised in that** a defined spacing of an occlusion opening in a desired occlusal position (7) of the guided bite guard (1) is determined automatically in a computer-aided manner or by a user.

6. The method according to any of claims 1-5, **characterised in that** at least one guide point (14, 15, 16) of the guide (3, 30, 31) to be constituted on the opposing jaw (4) is defined automatically in a computer-aided manner or manually by a user.

7. The method according to claim 6, **characterised in that** at least one movement profile (19, 20, 21, 22, 23) is defined for each guide point (14, 15, 16).

8. The method according to claim 7, **characterised in that** the surface shape of the guide (3, 30, 31) is automatically calculated in a computer-aided manner according to the at least one movement profile (19, 20, 21, 22, 23).

9. The method according to claim 8, **characterised in that** for each guide point (14, 15, 16) at least two movement profiles (19, 20, 21, 22, 23) are defined in two different planes, wherein the surface shape of the guide (3, 30, 31) is interpolated between the two planes of the two movement profiles (19, 20, 21, 22, 23) in such a way that a smooth transition is produced.

10. The method according to any of claims 1-9, **characterised in that**, during the construction of the bite guard (1), the surface of the virtual 3D model (13) of the guided bite guard (1) is reduced to such an extent that no surface point of the 3D model (6) of the opposing jaw (4) is virtually penetrated as long as the opposing jaw (4) is moved along the at least one guide (3) within the virtual articulator model (8) within a defined range of motion.

11. The method according to any of claims 1-10, **characterised in that** the guided bite guard (1) is produced fully automatically according to the constructed 3D model (13) of the bite guard (1) by means of a subtractive production process, such as a CAM production machine (38), or by means of an additive production process, such as a 3D printer.

## Revendications

1. Procédé pour fabriquer une gouttière dentaire (1) guidée pour une mâchoire d'appui (2) comprenant au moins un guide (3, 30, 31) pour une mâchoire opposée (4), dans lequel un modèle 3D (5) d'une mâchoire supérieure (2) et un modèle 3D (6) d'une mâchoire inférieure (4) sont présents, les modèles 3D (5, 6) de la mâchoire supérieure et de la mâchoire inférieure étant disposés dans une position occlusale (7) l'un par rapport à l'autre, un modèle 3D virtuel (13) de la gouttière dentaire (1) étant construit au moyen du modèle 3D (5) de la mâchoire supérieure (2) et du modèle 3D (6) de la mâchoire inférieure (4), l'au moins un guide (3, 30, 31) pour la mâchoire opposée (4) étant construit automatiquement par ordinateur sur le modèle 3D (13) de la gouttière dentaire (1), **caractérisé en ce que** le modèle 3D (13) de la gouttière dentaire (1) guidée est calculé automatiquement par ordinateur, les conditions à remplir étant que la gouttière dentaire (1) guidée couvre une zone d'appui définie sur la mâchoire d'appui (2), que, pour au moins une dent (17) de la mâchoire opposée (4), au moins une pointe de cuspide locale soit définie comme point d'appui (18).

2. Procédé selon la revendication 1, **caractérisé en ce que** les modèles 3D (5, 6) de la mâchoire supérieure et de la mâchoire inférieure sont intégrés dans un modèle d'articulateur virtuel (8) qui simule un mouvement d'articulation (10, 11, 12) de la mâchoire inférieure (4) par rapport à la mâchoire supérieure (2).

3. Procédé selon la revendication 1, **caractérisé en ce que** l'expansion de la gouttière dentaire (1) guidée sur la mâchoire supérieure (2) ou sur la mâchoire inférieure en tant que mâchoire d'appui (2) est déterminée automatiquement par ordinateur ou par un utilisateur.

4. Procédé selon l'une des revendications 1 à 3, **caractérisé en ce qu'**une épaisseur minimale de la gouttière dentaire (1) guidée est déterminée automatiquement par ordinateur ou par un utilisateur.

5. Procédé selon l'une des revendications 1 à 4, **caractérisé en ce qu'**un espacement défini d'une ouverture d'occlusion dans une position occlusale (7) souhaitée de la gouttière dentaire (1) guidée est déterminé automatiquement par ordinateur ou par un utilisateur.

6. Procédé selon l'une des revendications 1 à 5, **caractérisé en ce qu'**au moins un point de guidage (14, 15, 16) du guide (3, 30, 31) à constituer sur la mâchoire opposée (4) est défini automatiquement par ordinateur ou manuellement par un utilisateur.

7. Procédé selon la revendication 6, **caractérisé en ce qu'**au moins un profil de déplacement (19, 20, 21, 22, 23) est défini pour chaque point de guidage (14, 15, 16).

8. Procédé selon la revendication 7, **caractérisé en ce que** la forme de surface du guide (3, 30, 31) est calculée automatiquement par ordinateur en fonction de l'au moins un profil de déplacement (19, 20, 21, 22, 23).

9. Procédé selon la revendication 8, **caractérisé en ce que** pour chaque point de guidage (14, 15, 16) au moins deux profils de déplacement (19, 20, 21, 22, 23) sont définis dans deux plans différents, la forme de surface du guide (3, 30, 31) étant interpolé entre les deux plans des deux profils de déplacement (19, 20, 21, 22, 23) de manière à produire une transition lisse.

10. Procédé selon l'une des revendications 1 à 9, **caractérisé en ce que** lors de la construction de la gouttière dentaire (1), la surface du modèle 3D virtuel (13) de la gouttière dentaire (1) guidée est réduite à tel point qu'aucun point de surface du modèle 3D (6) de la mâchoire opposée (4) ne soit traversé virtuellement aussi longtemps que la mâchoire opposée (4) est déplacée le long de l'au moins un guide (3) à l'intérieur du modèle d'articulateur virtuel (8) dans une plage définie de mouvement.

11. Procédé selon l'une des revendications 1 à 10, **caractérisé en ce que** la gouttière dentaire (1) guidée selon le modèle 3D construit (13) de la gouttière dentaire (1) est fabriquée de façon entièrement automatique au moyen d'un procédé de fabrication par enlèvement de matière, tel qu'une machine de fabrication FAO (38), ou au moyen d'un procédé de fabrication additive, tel qu'une imprimante 3D.
